(19) Europäisches Patentamt
European Patent Office
Office européen des brevets

(11) Publication number: 0 323 540
A1

(12) # EUROPEAN PATENT APPLICATION

(21) Application number: 88100199.4

(22) Date of filing: 08.01.88

(51) Int. Cl.4: C07D 209/48 , C07D 209/76 , C07D 207/44 , C07D 519/00 , C08J 5/24 , H01B 1/12

(43) Date of publication of application: 12.07.89 Bulletin 89/28

(84) Designated Contracting States: CH DE ES FR GB IT LI SE

(71) Applicant: THE BOEING COMPANY
P.O. Box 3999, M.S. 85-74
Seattle Washington 98124(US)

(72) Inventor: Lubowitz, Hyman R.
26 Coral Tree Lane
Rolling Hills Estates, California(US)
Inventor: Sheppard, Clyde H.
12806 S.E. 45th Place
Bellevue, Washington 98006(US)
Inventor: Torre, Larry P.
1015 Bayshore Drive,Apt. 1009
Madison AL,35758(US)

(74) Representative: Baillie, Iain Cameron et al
c/o Ladas & Parry Isartorplatz 5
D-8000 München 2(DE)

(54) **Conductive, thermally stable oligomers.**

(57) The morphology of multidimensional, thermally stable oligomers is combined with the inclusion of charge carrier linkages within the polymer arms to produce thermally stable advanced composites from cured oligomers that are conductive or semiconductive if suitably doped.

EP 0 323 540 A1

Xerox Copy Centre

# CONDUCTIVE, THERMALLY STABLE OLIGOMERS

## TECHNICAL FIELD AND BACKGROUND OF THE INVENTION

The present invention relates to a family of thermally stable oligomers that are conductive or semiconductive when suitably doped. Aromatic polymeric arms containing conductive linkages project from a central aromatic hub and end with crosslinking end groups. When the oligomer is cured, three-dimensional polymeric matrices are formed with controlled crosslinking to achieve high performance composites.

While epoxy-based composites are suitable for many applications, their brittle nature and susceptibility to degradation make them inadequate for many aerospace applications, especially those applications which require thermally stable, tough composites. Accordingly, research has recently focused upon polyimide composites to achieve an acceptable balance between thermal stability, solvent resistance, and toughness. Still the maximum temperatures for use of the polyimide composites, such as PMR-15, are about 600-625° F, since they have glass transition temperatures of about 690° F.

Polybenzoxazoles may be used at temperatures up to about 750-775° F, since these composites have glass transition temperatures of about 840° F. Aerospace applications need composites which have even higher use temperatures while maintaining toughness, solvent resistance, processibility, formability, strength, and impact resistance.

Chemists have sought to synthesize these new oligomers for high performance advanced composites, resulting in a progression of compounds synthesized to provide unique properties or combinations of properties. For example, Kwiatkowski and Brode disclosed maleic capped linear polyarylimides in U.S. Patent 3,839,287. Holub and Evans disclosed maleic or nadic capped imido-substituted polyester compositions in U.S. Patent 3,729,446. Lubowitz and Sheppard disclosed thermally stable polysulfone oligomers in U.S. Patent 4,476,184, and have continued to make advances with polyetherimidesulfones, polybenzoxzolesulfones, and polybutadienesulfones. "Star" and "star-burst" multidimensional oligomers exhibit surprisingly high glass transition temperatures. The multidimensional oligomers have superior processibility than some advanced composite oligomers since they can be handled at lower temperatures. Upon curing, however, the thermal resistance of the resulting composite is markedly increased with only a minor loss of stiffness, matrix stress transfer (impact resistance), toughness, elasticity, and other mechanical properties.

While polyesters are among the most highly developed polymers, commercial polyesters do not exhibit satisfactory thermal and oxidative resistance to be useful for aircraft or aerospace applications. Even polyarylesters are unsatisfactory, since the resins are generally insoluble in laminating solvents, are intractable in fusion, and shrink or warp during composite fabrication. The high concentration of ester groups contributes to resin strength and tenacity, but also makes the resin susceptible to the damaging effects of water absorption. High moisture absorption can lead to distortion of the composite when it is loaded at elevated temperature.

Conductive and semiconductive plastics have been extensively studied (see, e.g., U.S. Patents 4,375,427; 4,338,222; 3,966,987; 4,344,869; and 4,344,870). These prior art polymers do not possess the blend of properties which are essential for aerospace applications. That is, the conductive polymers do not possess the blend of (1) toughness, (2) stiffness, (3) elasticity, (4) processibility, (5) impact resistance and other matrix stress transfer capabilities, (6) retention of properties over a broad range of temperatures, and (7) high temperature resistance which is desirable on aerospace advance composites. The prior art composites are often too brittle.

High performance, aerospace advanced composites can be prepared using crosslinkable, end capped polyester imide either sulfone oligomers that have the desired combined properties of solvent resistance, toughness, impact resistance, strength, processibility, formability, and thermal resistance. By including Schiff base (-C=N-), imidazole, thiazole, or oxazole linkages in the oligomer chain, the linear, advanced composites formed with oligomers can have semiconductive or conductive properties when appropriately doped.

Glass transition temperatures above 900° F are achievable with multidimensional, branched oligomers which include crosslinking groups at the ends of linear aromatic "sulfone" arms radiating from an aromatic hub. When cured to crosslink the end caps, these "star" and "star-burst" oligomers provide a multidimensional array. The resulting thermally stable composite exhibits excellent toughness and processibility. The crosslinking groups (usually nadic and acetylenic phenylimide moieties) also provide solvent resistance to the composites. Glass transition temperatures of about 950° F or higher are achievable.

The present invention combines features of the "Schiff base" conductive sulfone polyarylesters with the

"star" and "star-burst" multidimensional morphology to create advanced conductive composites.

SUMMARY OF THE INVENTION

According to one aspect of the invention, there is provided an oligomer that is conductive or semiconductive when suitably doped with a conventional dopant, comprising an aromatic hub having at least three arms radiating from the hub, each arm being essentially a polyaryl chain including at least one conductive linkage selected from the group consisting of Schiff bases (-CH=N-), oxazoles, thiazoles, and imidazoles and including terminal crosslinking end caps selected from the group consisting of:

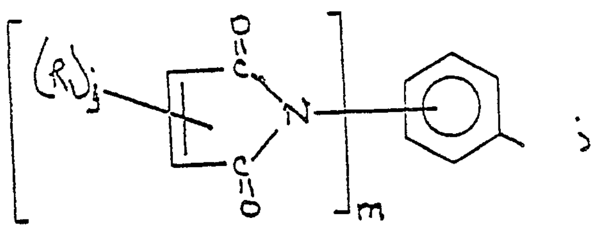

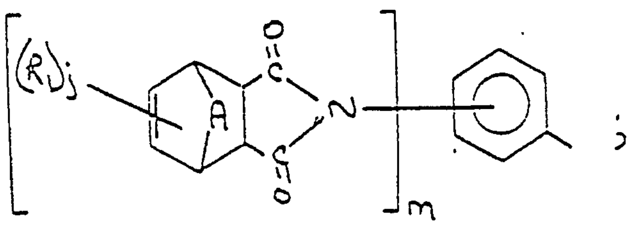

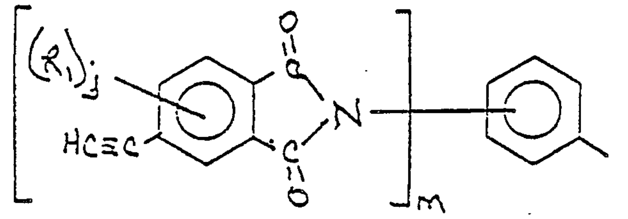

;

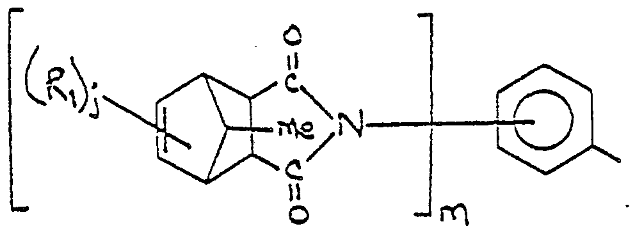

; and

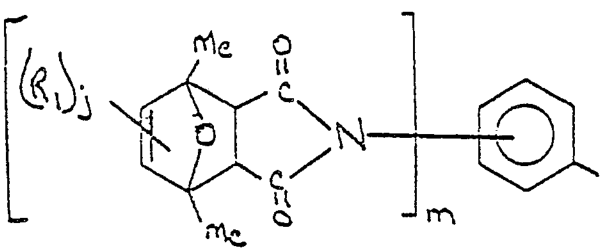

wherein m = 1 or 2;

$R_1$ = lower alkyl, lower alkoxy, aryl, and substituted aryl;

A = $-CH_2-$, -S-, -O-, or $-SO_2-$; and

j = 0, 1, or 2.

The commercially available conductive or semiconductive polymers that are usually intractable and infusible. They also degrade at temperatures above about 100° C, and, accordingly, are unsatisfactory for aerospace 'applications. Linear "Schiff base" polyesters may have poor solubility and poor melt-flow characteristics, which make them difficult to process into composites. Therefore the present invention combines (1) the morphology behind the thermally stable, "star" or "star-burst" oligomers with (2) relatively short polymeric arms of polyester or polyether "Schiff base" compounds and with (3) crosslinking end caps to provide, upon doping, conductive or semiconductive oligomers with high use temperatures.

These preferred compounds generally are made by reacting an aromatic hub, such as a compound of the general formula:

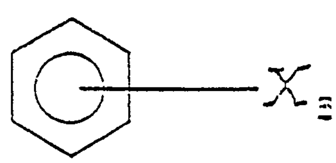

wherein x = -CHO, -OH, $-NH_2$, $-\overset{O}{\overset{\|}{C}}-Cl$, or halogen, with a corresponding mono- or difunctional crosslinkable end cap of the general

formula:

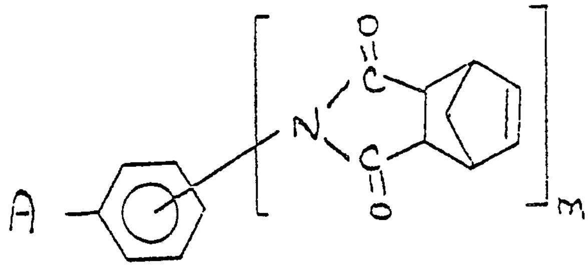

wherein A = X as defined for the hub, provided that if X -CHO, $A = -\overset{O}{\overset{\|}{C}}-Cl$; and m = 1 or 2, and with the appropriate mixtures of diamines, aldehydes, and phenols to achieve "Schiff base" arms radiating from the hub.

Preferred products have the general formula:

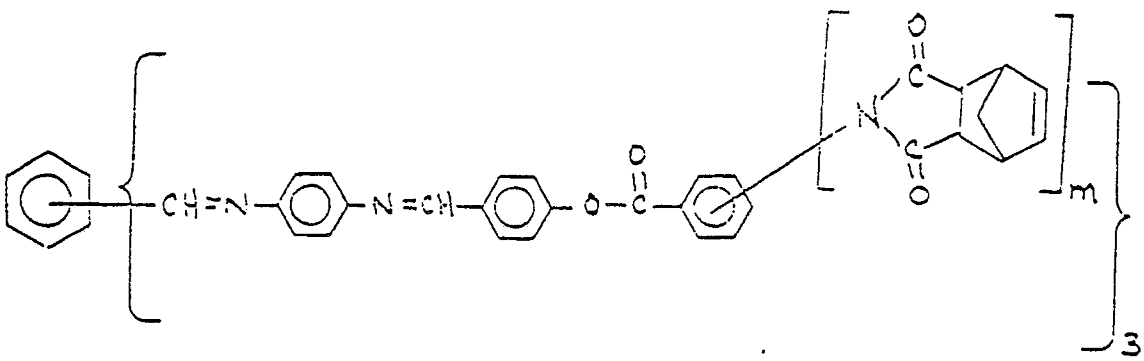

or

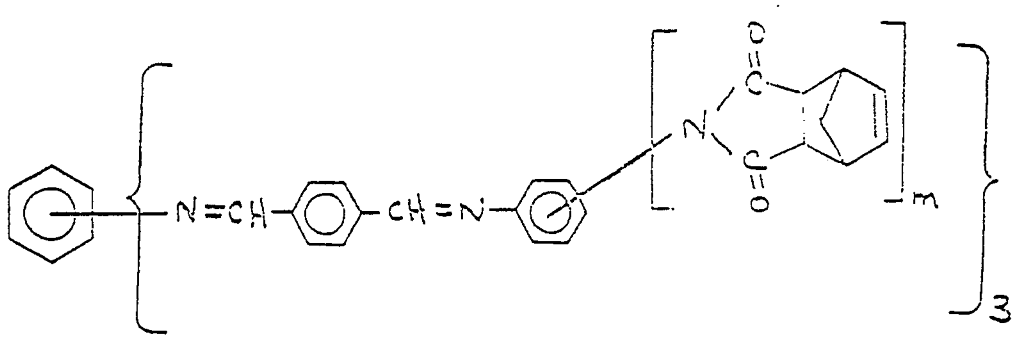

of course, oligomers with more arms or longer arms might also be made.

## DETAILED DESCRIPTION OF THE INVENTION

Thermally stable oligomers having semiconductive or conductive properties when doped with suitable dopants have a multidimensional oligomer morphology. The linear arms of the oligomers contain Schiff base linkages (or other conductive linkages) between aromatic groups. Sulfone and ether linkages may be interspersed in the arms, as will be explained. Each arm is terminated with a mono- or difunctional end cap to allow controlled crosslinking upon heat-induced or chemically-induced curing. The resulting oligomers

and composites are conductive, thermally stable compounds suitable for many aerospace applications. For example,

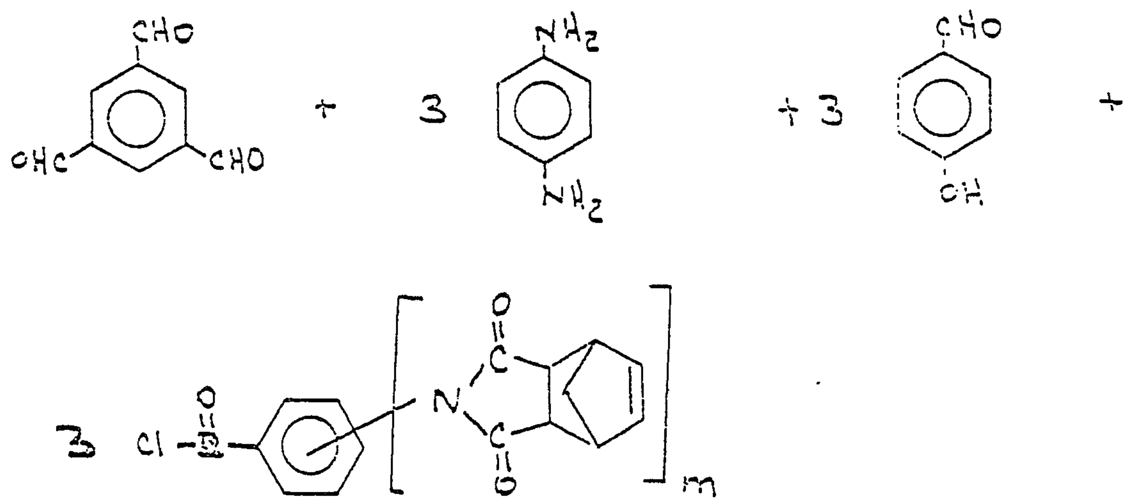

can be mixed and reacted under an inert atmosphere to yield:

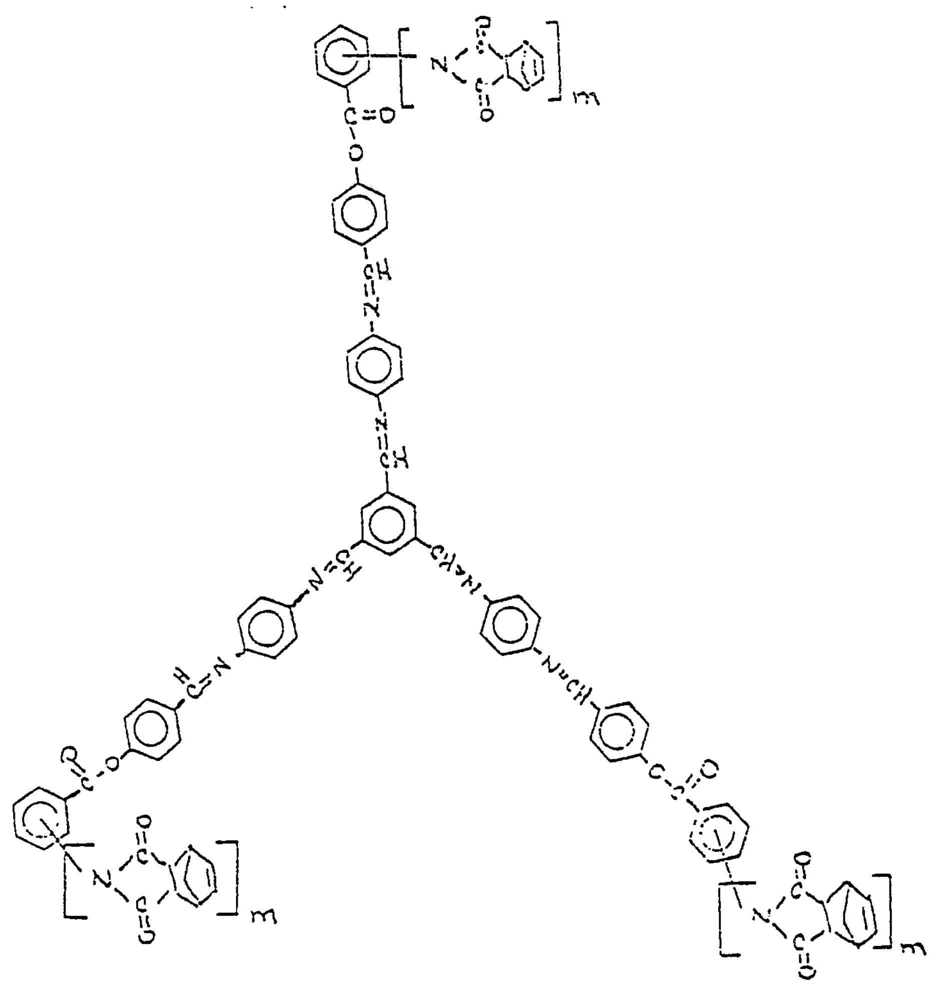

wherein m = 1 or 2.

In general terms, the invention relates to a process and the products of the process for making "star" (m = 1) or "star-burst" (m = 2) multidimensional oligomers of this general type.

Thermally stable oligomers suitable for high temperature advanced composites are synthesized to include a high degree of aromatic groups. The stable aromatic bond energies allow synthesis of an oligomer with outstanding thermal stability. Acceptable toughness and impact resistance is gained through electronegative linkages within the linear chains of aromatic groups that radiate from the central aromatic hub. The electronegative linkages are generically called "sulfone" linkages for purposes of this description, and include the groups:

-CO-;

$-SO_2-$;

$-(CF_3)C-$; and

-S-.

Generally, -CO- and $-SO_2-$ groups are preferred for cost, convenience, and performance. The group -S-S- should be avoided, since it is too thermally labile.

The preferred aromatic moieties are aryl groups, such as phenyl, biphenyl and naphthyl. Other aromatic groups can be used, if desired, since their stabilized aromatic bonds should provide the desired thermal stability. For example, azaline groups may be used. The aryl groups may include substituents, if desired, such as halogen, lower alkyl up to about 4 carbon atoms, lower alkoxy up to about 4 carbon atoms, or aryl side chains. Steric hindrance may arise in synthesizing the oligomers or in crosslinking the oligomers into the final composites, if the side chains are too large. The substituents may also effect the thermal stability of the resulting oligomers and composites. Unsubstituted phenyl groups are preferred for cost, convenience, and performance.

Improved performance and thermal stability is gained through the creation of a multidimensional, crosslinked polymer. An aromatic hub includes a plurality of rays or spokes radiating from the hub in the nature of a star to provide multidimensional crosslinking with a greater number of crosslinking bonds than linear arrays along through suitable terminal groups. Usually the hub will have three radiating chains to form a Y pattern. In some cases, four chains may be used. Including more chains leads to steric hindrance as the hub is too small to accommodate the radiating chains. A trisubstituted phenyl hub is highly preferred with the chains being symmetrically placed about the hub. Biphenyl, naphthyl, or azaline may also be used as the hub along with other aromatic moieties, if desired.

The chains include crosslinking end groups, which improve the solvent-resistance of the polymers and advanced composites, and which further stabilize the polymer. These end groups may be thermally or chemically activated during the curing step to provide a strongly crosslinked, complex, multidimensional array of interconnected oligomers. When the goal is an advanced composite having a glass transition temperature above 900° F (and preferably above 950° F) each end cap should have high thermal stability and a high thermal activation temperature. End caps with two crosslinking functionalities (difunctional end caps) are expected to yield the highest crosslinked arrays.

Although the para isomer is illustrated, other isomers may be used. The highest thermal stabilities appear to be achievable with unsubstituted phenyl chains of short length when these chains are capped with difunctional end caps.

Each arm of the oligomer includes a conductive linkage, such as a Schiff base (-CH=N-) linkage, and chain extender portions such as the single benzene groups illustrated in the example. By the proper combination of aldehydes, amines, alcohols (phenols), and acid chlorides, a family of oligomers can be made.

The oligomers may be formed by the attachment of arms to the hub followed by chain termination in two steps. For example, tribromobenzene may be mixed with p-aminophenol and 4,4,dibromodiphenylsulfone and reacted under an inert atmosphere at an elevated temperature to achieve an amino terminated "star" of the general formula:

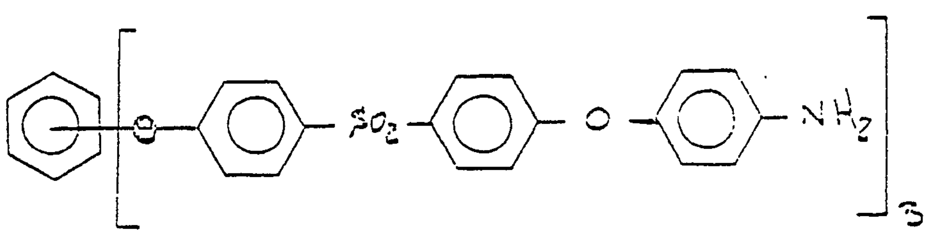

which can be reacted with suitable aldehydes, phenols, and end caps to yield a suitable end capped oligomer. Those skilled in the art will recognize the generality of this synthetic pathway to achieve "stars"

with extended conductive arms including phenoxy-phenyl sulfone linkages interspersed with the conductive linkages.

The oligomers can be synthesized in a homogeneous reaction scheme wherein all the reactants are mixed at one time, or in a stepwise reaction scheme wherein the radiating chains are affixed to the hub and the product of the first reaction is subsequently reacted with the end cap groups. Of course, the hub may be reacted with end-capped arms that include one reactive, terminal functionality of linking the arm to the hub. Homogeneous reaction is preferred, resulting undoubtedly in a mixture of oligomers because of the complexity of the reactions. The products of the processes (even without distillation or isolation of individual species) are preferred oligomer mixtures which can be used without further separation to form the desired advanced composites.

Oligomers can be synthesized from a mixture of four or more reactants so that extended chains may be formed. Adding components, however, adds to the complexity of the reaction and of its control. Undesirable competitive reactions may result or complex mixtures of macromolecules having widely different properties may be formed, because chain extenders and chain terminators are mixed, and compete against one another.

The selection of arms and end caps can effect the thermal stability, toughness, processibility, impact resistance, and solvent resistance of the resulting oligomers and advanced composites formed with the cured oligomers. Longer arms may result in reduced thermal stability since the relative proportion of crosslinking bonds will be reduced, and the crosslinking bonds will be spaced farther apart. Since the end caps exhibit different thermal properties, they will undoubtedly impart different properties to the resulting composite. Although the goal is advanced composites with thermal stabilities above 900° F, a wide variety of composites with different use ranges can be made, and are all considered to be within the class of compounds contemplated by this invention. To that end, the end caps may be selected from the group consisting of:

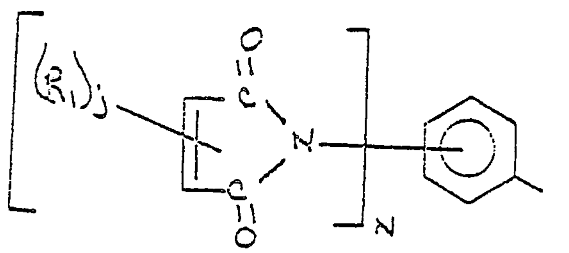

;

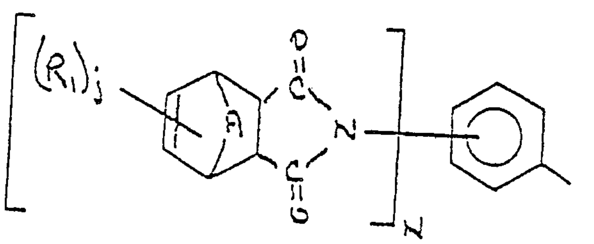

;

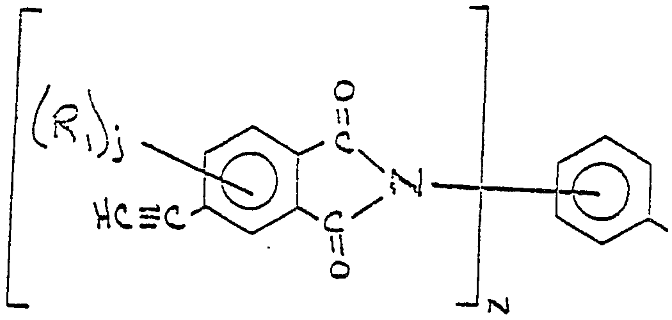

;

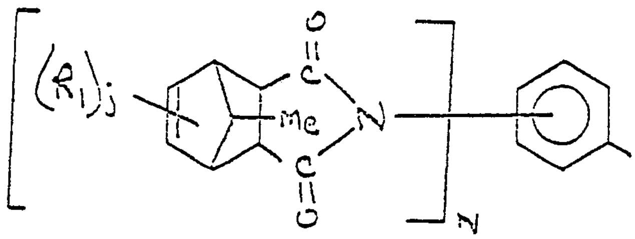

; and

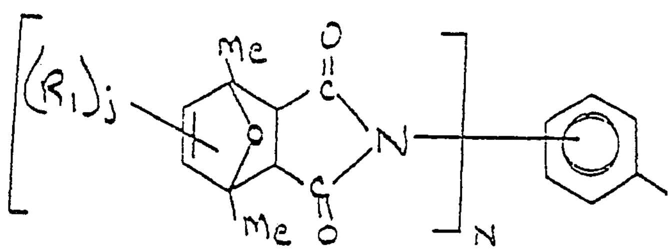

wherein n = 1 or 2;
$R_1$ = lower alkyl, lower alkoxy, aryl, and substituted aryl (preferably H- or $CH_3$-);
A = $-CH_2-$, -S-, -O-, or $-SO_2-$ (preferably $-CH_2$); and
j = 0, 1, or 2.

Maleic, dimethyl oxynadic, ethynyl, trimethylsilylethynyl, and phenylethynyl end groups may also be used, if desired. These end caps will probably allow curing at lower temperatures, and will probably produce composites of lower thermal stability.

To obtain the highest thermal stability in the oligomers and advanced composites, the preferred end caps are:

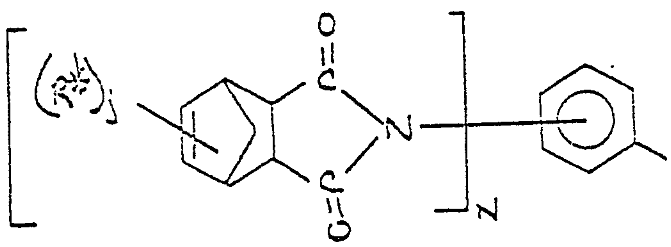

or

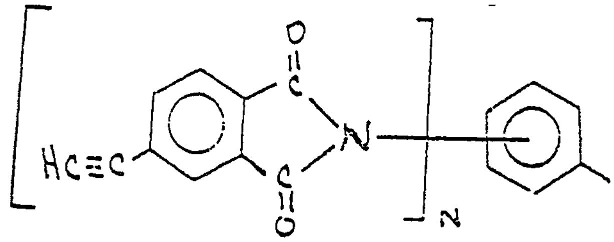

wherein R* = $CH_3$-; n = 1 or 2 (preferably 2); and j = 0, 1, or 2.

Although aryl backbones for the chains are preferred, aliphatic moieties, such as MCTC, may be incorporated into the arms if desired. Aliphatic moieties probably will reduce the thermal stability of the resulting composites since aliphatic bonds are less stable than aromatic bonds.

For purposes of this description, "sulfone" is generally used in a generic way to include $-SO_2-$(sulfone), -CO- (carbonyl), $-(CF_3)_2C$ (hexafluoroisopropanyl), or -S- (sulfide). Thus, for example, the $-SO_2-$ linkage of the amino terminated "star" can have any of the identified, electronegative linkages substituted for it in the family of oligomers contemplated by this invention.

"Schiff base" is also generally used in a generic way to represent conductive linkages such as -CH=N-, oxazoles, thiazoles, or imidazoles.

Dopants for creating semiconductive or conductive composites are preferably selected from compounds commonly used to dope other polymers, namely (1) dispersions of alkali metals (for high activity) or (2) strong chemical oxidizers, particularly alkali perchlorates (for lower activity). Arsenic compounds and elemental halogens, while active dopants, are too dangerous for general usage, and are not recommended.

The dopants react with the polymers to form charge transfer complexes. N-type semiconductors result from doping with alkali metal dispersions. P-type semiconductive result from doping with elemental iodine or perchlorates.

While research into conductive or semiconductive polymers has been intense, the resulting compounds (mainly polyacetylenes, polyphenelenes, and polyvinylacetylenes) are unsatisfactory for aerospace applications because the polymers are:

(a) unstable in air;
(b) unstable at high temperatures;
(c) brittle after doping;
(d) toxic because of the dopants; or
(e) intractable.

These problems are overcome or significantly reduced with the conductive oligomers of the present invention.

The polymers of the present invention generally exhibit greater oxidative stability and greater dimensional stability at elevated temperatures, greater impact strengths, greater dimensional stiffness over a wider range of temperatures, and greater toughness than prior art conductive oligomers and composites.

While conventional theory holds that semiconductive polymers should have (1) low ionization potentials, (2) long conjugation lengths, and (3) planar backbones, there is an inherent trade-off between conductivity and toughness or processibility, if these constraints are followed. To overcome the processing and toughness shortcomings common with Schiff base, oxazole, imidazole, or thiazole polymers, the oligomers of the present invention, include "sulfone" linkages interspersed along the backbone providing a mechanical swivel for the rigid, conductive segments of the arms. Phenoxyphenylsulfone or phenoxyphenylketone

moieties are preferred. The resulting compounds are polyphenoxyphenylsulfoneimido polymers with conductive segments.

Preferred Schiff base segments for the arms have the general formula:

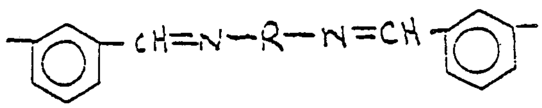 ;

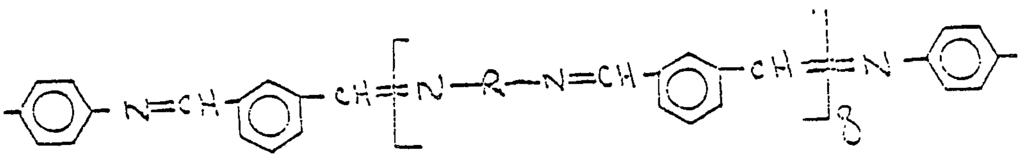 ; OR

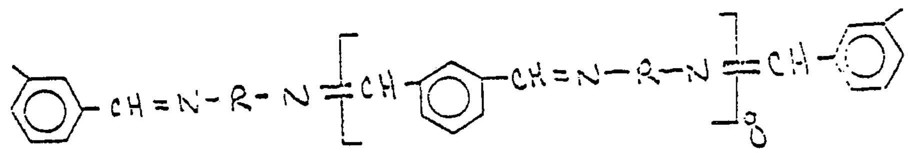

wherein R = an aromatic moiety or a short aryl chain including a plurality of aryl moieties linked with any of $-CH_2-$, $-SO_2-$, -S-, _O-, -CO-, $-(CH_3)_2C-$, or $-(CF_3)_2C-$and q = 0 4. R is generally selected from the group consisting of:
phenyl;
biphenyl;
naphthyl; or
a compound of the general formula:

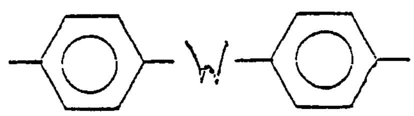

wherein W = $-CH_2-$ or $-SO_2-$.

Similar segments include oxazole

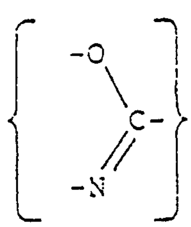 ,

thiazole

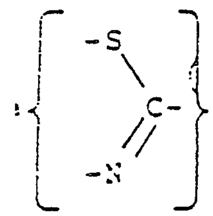,

or imidazole

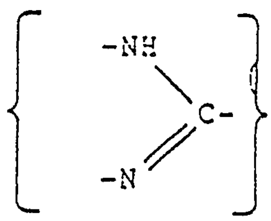

linkages instead of (-CH=N-) linkages can also be used. Benzoxazoles, benzthiazoles, and benzimidzoles may be used as "Schiff base" linkages.

Because it may be difficult to make precursor molecules having intermediate benzimidazole, benzoxazole, or benzthiazole linkages, and because the chemistry for Schiff base (-CH=N-) compounds is well understood, it is preferred to use Schiff base segments in the oligomers for semiconductive or conductive applications.

Solubility of the oligomers becomes an increasing problem as the length of the chains increases. Therefore, shorter chains are preferred, if the resulting oligomers remain processible. That is, the chains should be long enough to yield thermoplastic characteristics but short enough to keep the oligomers soluble during the reaction sequence.

The reactive aldehydes, diamines, alcohols (phenols), or acid halides may have backbones of the following general nature and may be combined in many ways to form oligomers of the general class of interest.

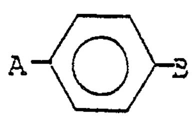

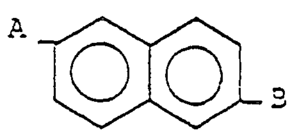

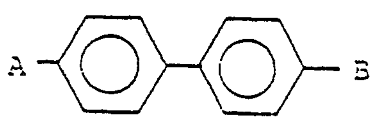

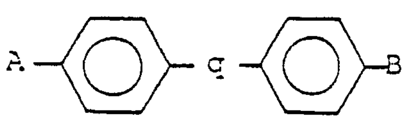

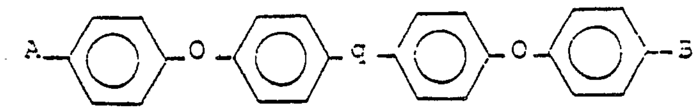

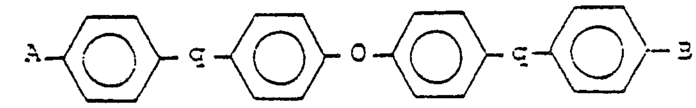

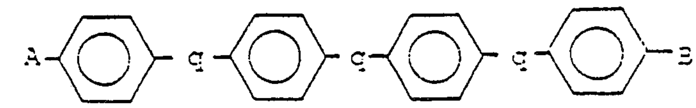

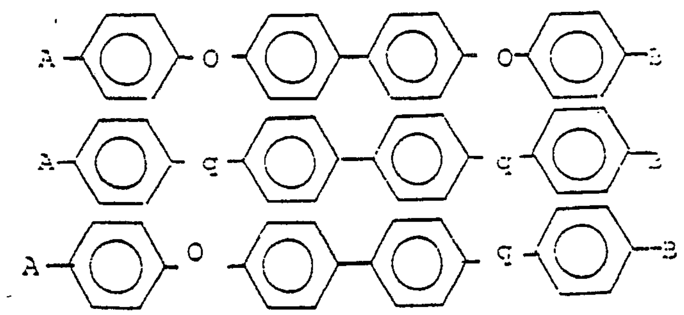

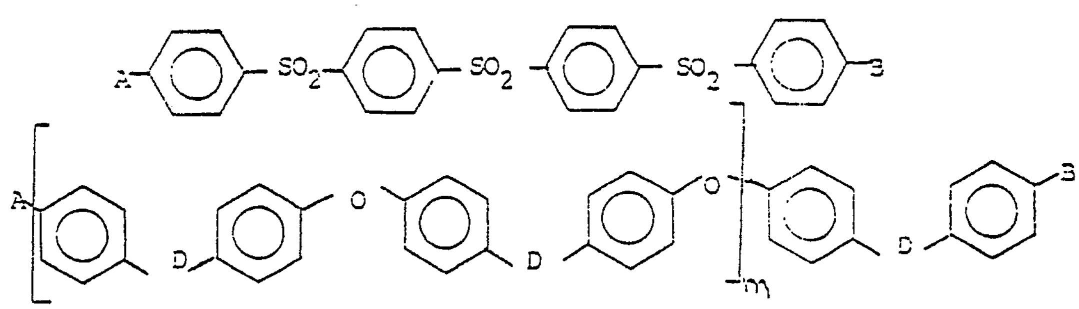

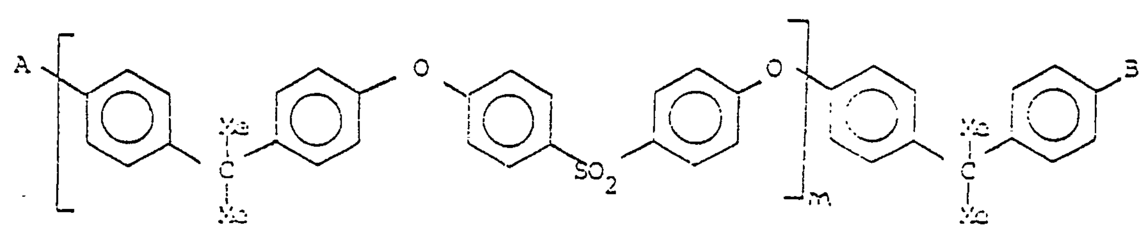

wherein q = $-SO_2-$, -CO-, -S-, or $-(CF_3)_2C-$, and preferably $-SO_2-$ or -CO-;
Me = $CH_3-$;
m = an integer, generally less than 5, and preferably 0 or 1;
D = any of -CO-, $-SO_2-$, or $-(CF_3)_2C-$;
A = -CHO, $-NH_2$, -OH, or

$$-\overset{\overset{O}{\|}}{C}-X;$$

B = -CHO, $-NH_2$, -OH, or

$-\overset{O}{\overset{\parallel}{C}}-X$, such that if A = -OH, B = -CHO and that if B = -OH, A = -CHO; and X = halogen.

## EXAMPLE 1

A preferred oligomer is prepared by reacting:

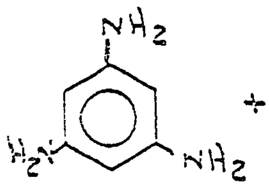
+ 3
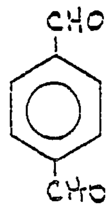
+ 3
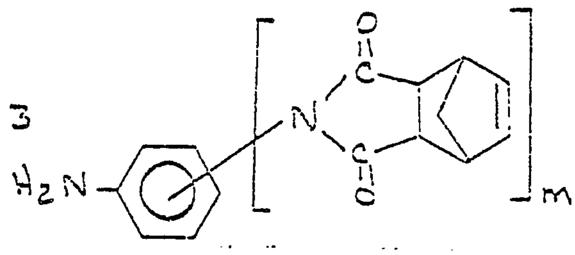

under an inert atmosphere to yield:

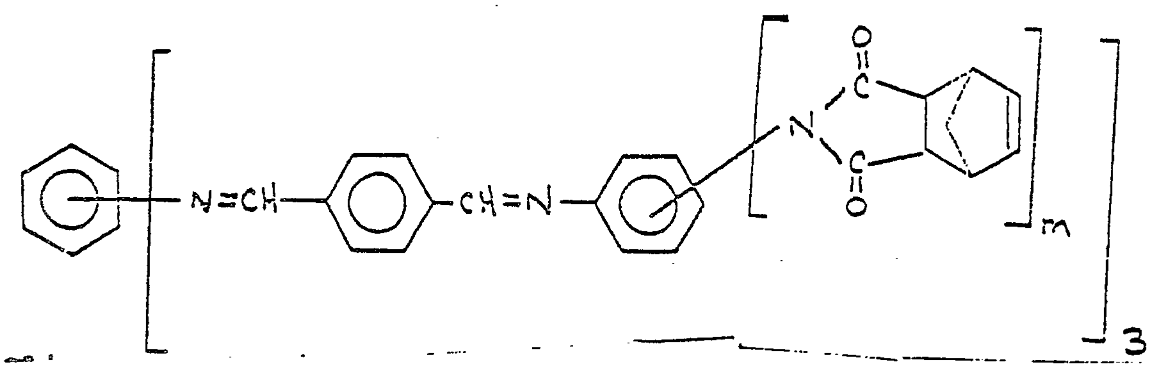

Oligomers of this general type are preferred because the starting materials are easily obtained and are inexpensive.

## EXAMPLE 2

Another preferred oligomer is prepared by reacting:

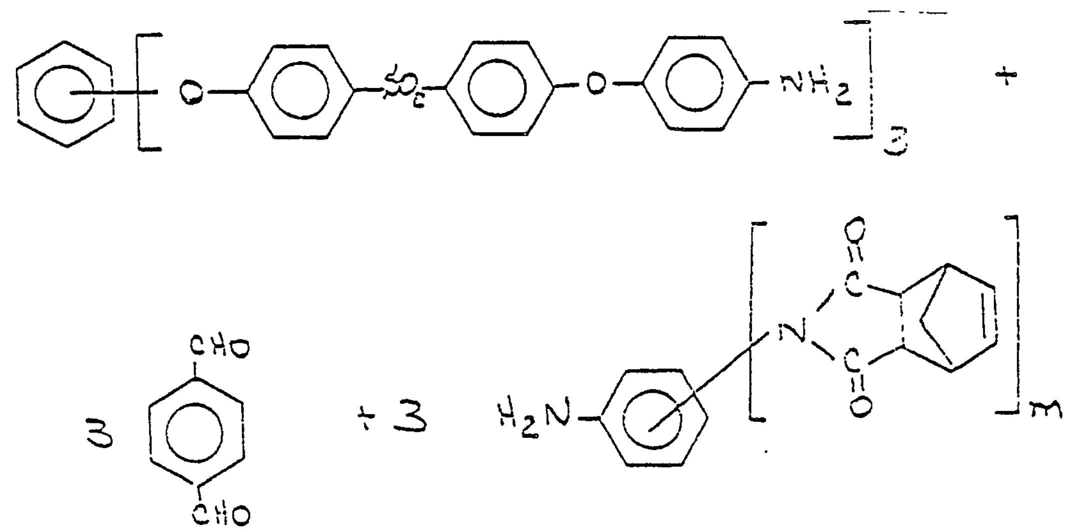

under an inert atmosphere to yield:

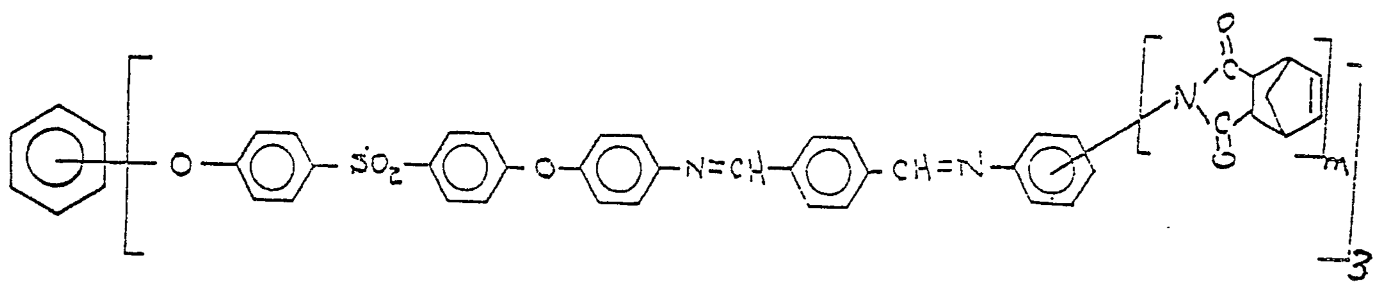

EXAMPLE 3

Another preferred oligomer is prepared by reacting:

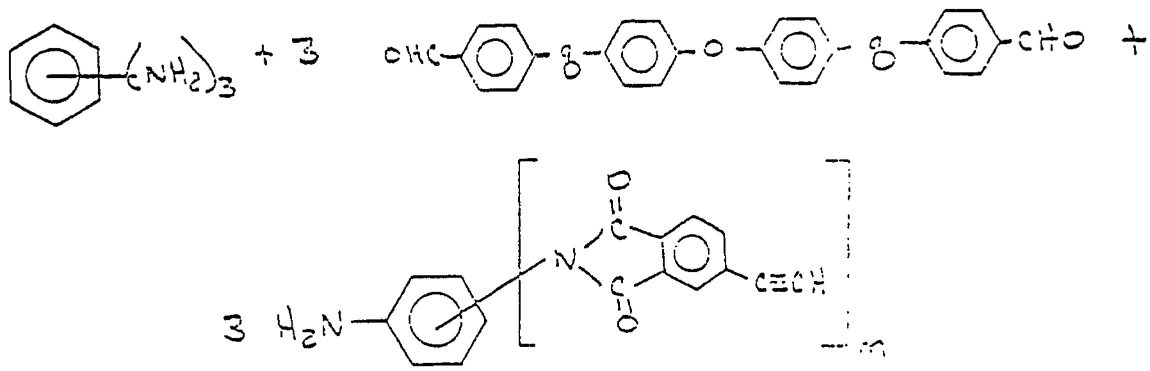

under an inert atmosphere to yield:

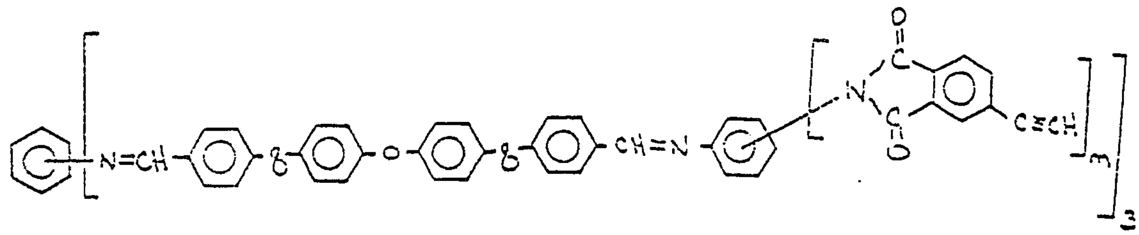

wherein q = $-SO_2-$, -CO-, -S-, or $-(CF_3)_2C-$, and preferably $-SO_2-$ or -CO-

## EXAMPLE 4

Another preferred oligomer is prepared by reacting:

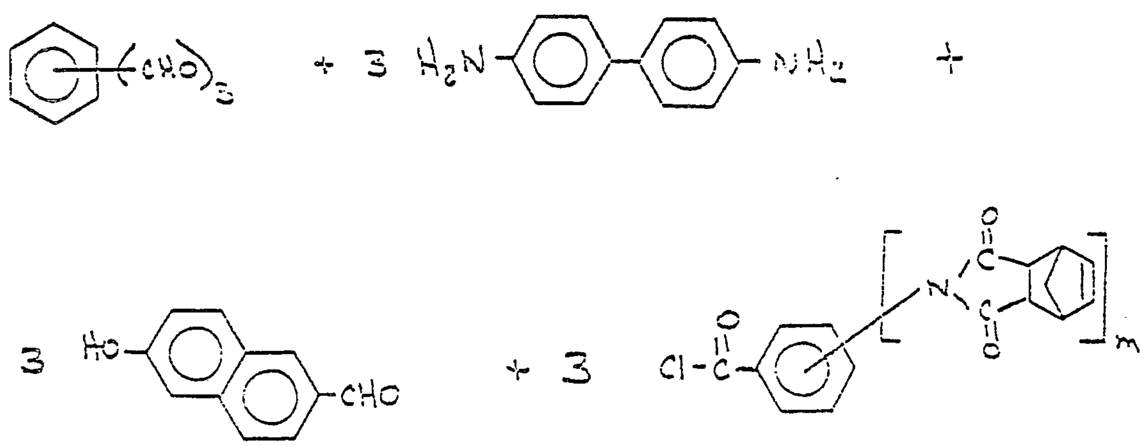

under an inert atmosphere to yield:

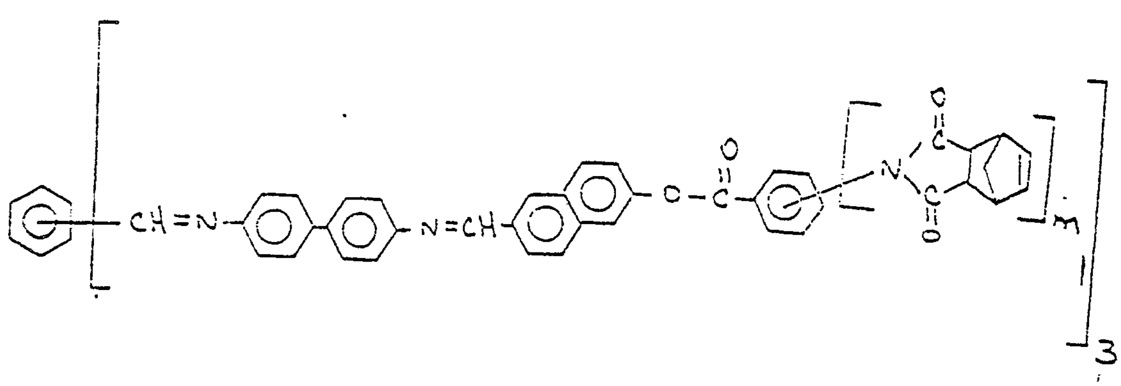

## EXAMPLE 5

Yet another preferred oligomer is prepared by reacting:

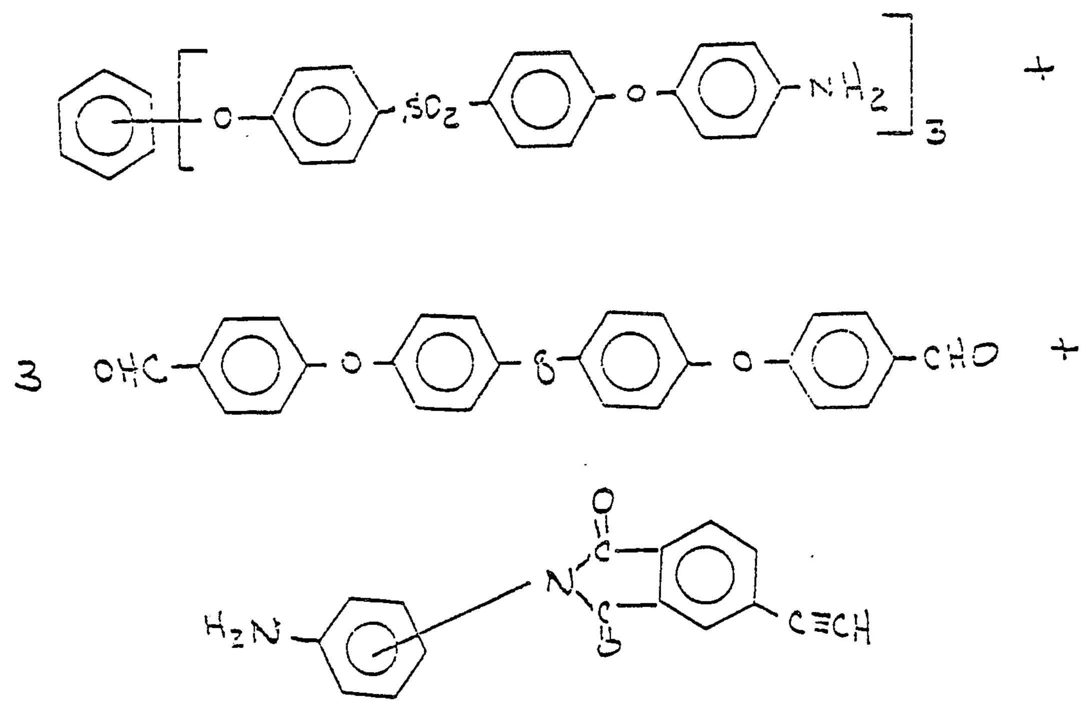

under an inert atmosphere to yield:

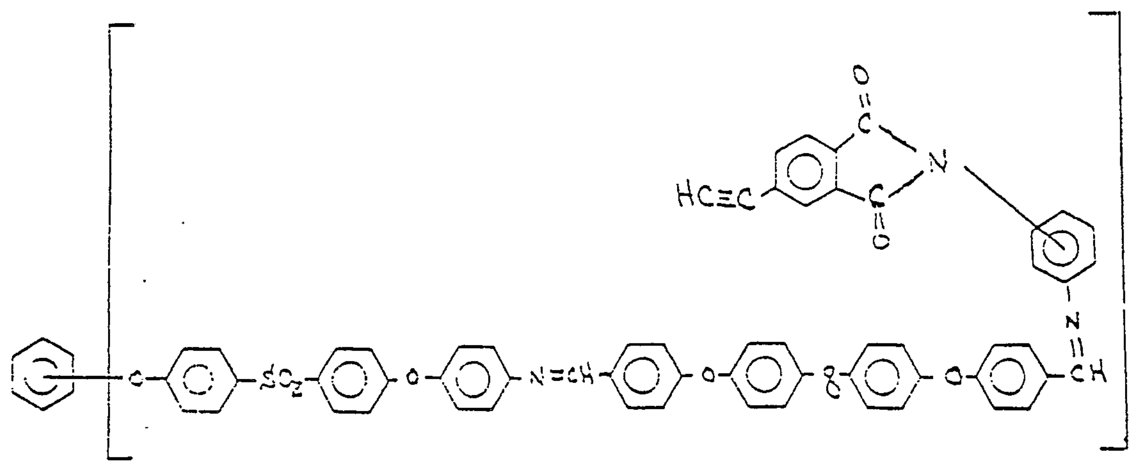

wherein q = $-SO_2-$, -CO-, -S-, or $-(CF_3)_2C-$, and preferably $-SO_2-$ or -CO-.

From these hypothetical examples, those skilled in the art will recognize the generality of the synthesis and will recognize the family of oligomers contemplated by the invention.

While the arms should be polyaryl, linear chains to obtain the highest thermal stability, such stability is only required for certain aerospace applications of composites formed with the oligomers. Accordingly, the invention also contemplates a family of semiconductive or conductive oligomers of lower thermal stability where, the arms may include saturated or unsaturated aliphatic groups, such as MCTC, between the conductive linkages. Because aliphatic bonds have lower bond energies than aromatic bonds, oligomers of this general type are likely to have lower thermal stability. Use of only aromatic groups between linkages is preferred.

Prepregs and advanced composites can be readily prepared from the oligomers by conventional techniques. For example, the oligomers can be applied to a fiber cloth reinforcement, and the resulting prepreg can be cured in a vacuum bag process at an elevated temperature. The dopant should be added to the oligomer prior to prepregging.

While preferred embodiments have been described, those skilled in the art will readily recognize

alterations, variations, and modifications which might be made without departing from the inventive concept. Therefore, the claims should be interpreted liberally with the support of the full range of equivalents known to those of ordinary skill based upon this description. The examples are given to illustrate the invention and not to limit it. The claims should be limited only as is necessary in view of the pertinent prior art.

## Claims

1. An oligomer that is conductive or semiconductive when suitably doped with a conventional dopant, comprising an aromatic hub having at least three arms radiating from the hub, each arm being essentially a polyaryl chain including at least one conductive linkage selected from the group consisting of Schiff bases (-CH = N-), oxazoles, thiazoles, and imidazoles and including terminal crosslinking end caps selected from the group consisting of:

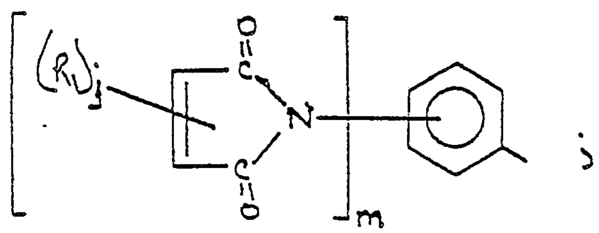

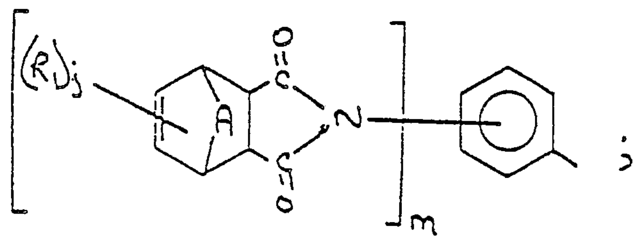

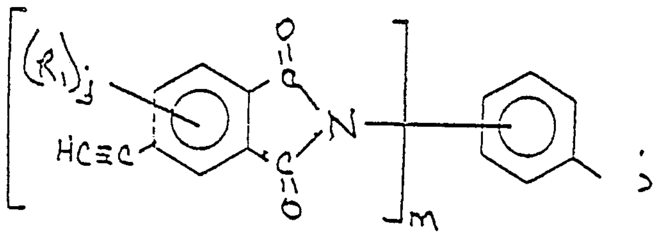

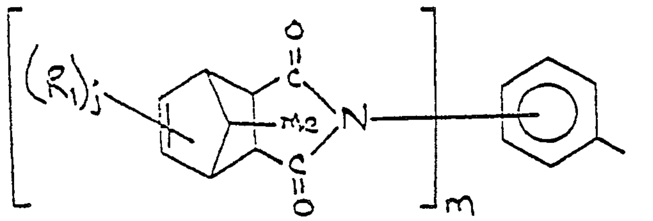 ; and

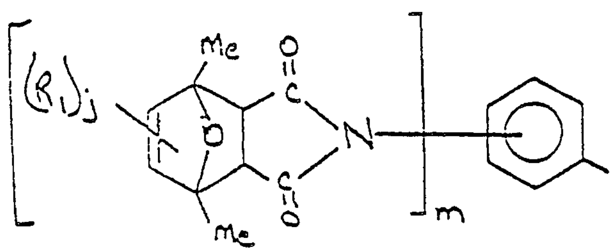

wherein m = 1 or 2;
$R_1$ = lower alkyl, lower alkoxy, aryl, and substituted aryl;
A = $-CH_2-$, -S-, -O-, or $-SO_2-$; and
j = 0, 1,or 2.

2. The oligomer of claim 1 wherein the conductive linkage is a Schiff base.

3. The oligomer of claim 1 wherein the conductive linkage is selected from the group consisting of benzoxazoles, benximidazoles, and benzthiazoles.

4. The oligomer of claim 1 wherein the hub is selected from the group consisting of phenyl, naphthyl, biphenyl, and azalinyl.

5. The oligomer of claim 2 wherein the hub is selected from the group consisting of phenyl, naphthyl, biphenyl, and azalinyl.

6. The oligomer of claim 1 wherein the hub is phenyl.

7. The oligomer of claim 2 wherein the hub is phenyl.

8. The oligomer of claim 1 wherein the oligomer has three arms.

9. The oligomer of claim 1 wherein the end cap is selected from the group consisting of:

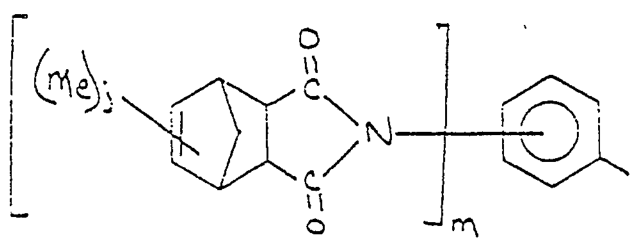 or

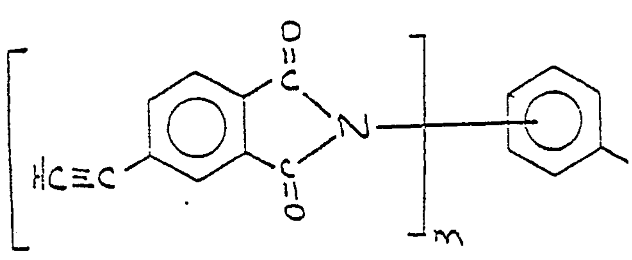

wherein Me = $-CH_3-$; m = 1 or 2; and j = 0, 1, or 2.

10. The oligomer of claim 8 having a structural formula selected from the group consisting of:

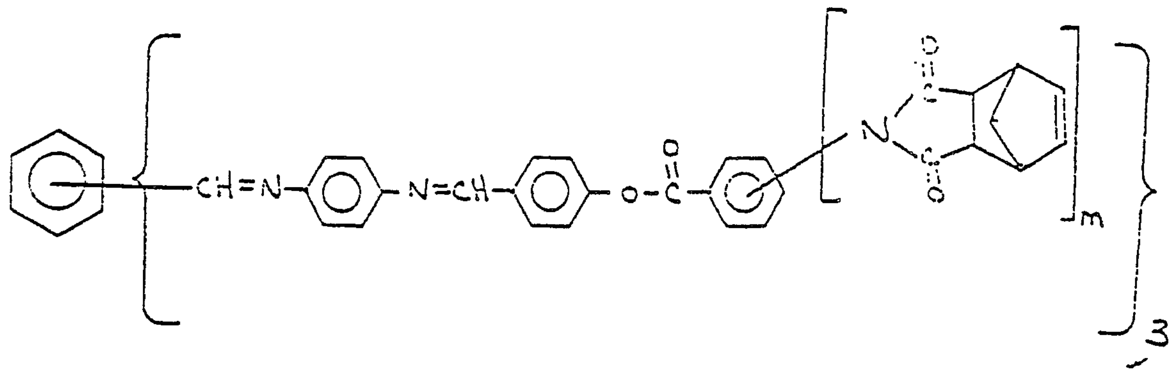

or

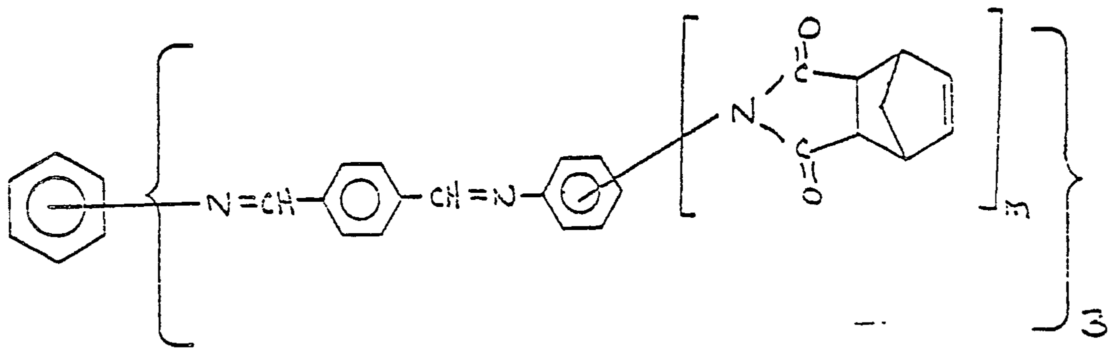

wherein m = 1 or 2.

11. The oligomer of claim 8 having the structural formula:

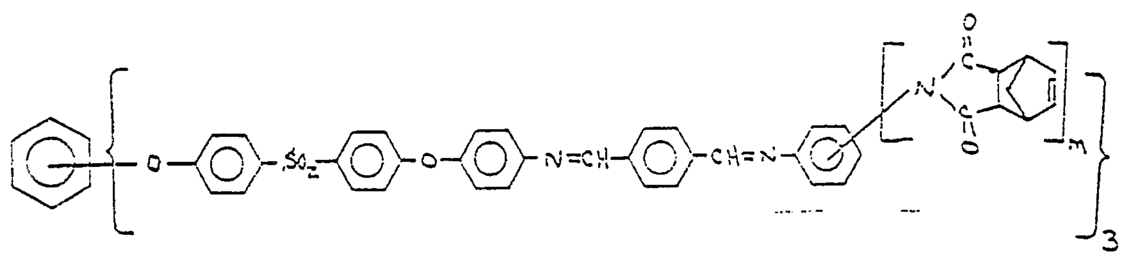

12. A method for making an oligomer that is conductive or semiconductive when suitably doped with a conventional dopant, comprising the steps of:
reacting one mole of an aromatic hub of the general formula:
$Ar\text{-}(X)_k$,
wherein Ar = an aromatic compound;
X = CHO, -OH, $-NH_2$,
$-\overset{O}{\overset{\|}{C}}$-CL, or halogen
k = 3 or 4, with at least three moles of a corresponding mono- or difunctional crosslinkable end cap selected from the group of compounds having the general formulae:

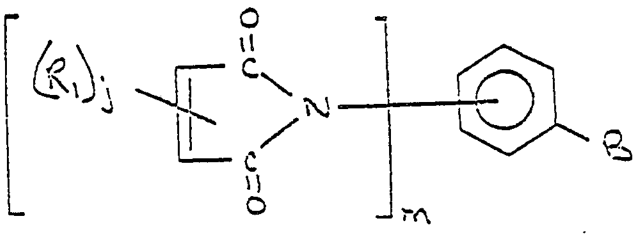

;

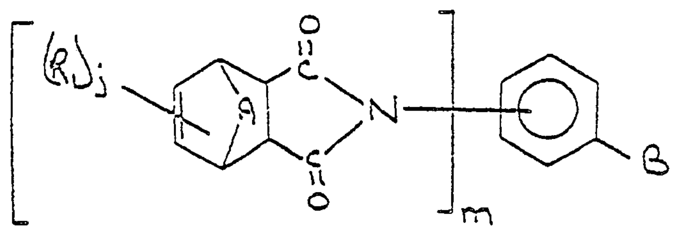

;

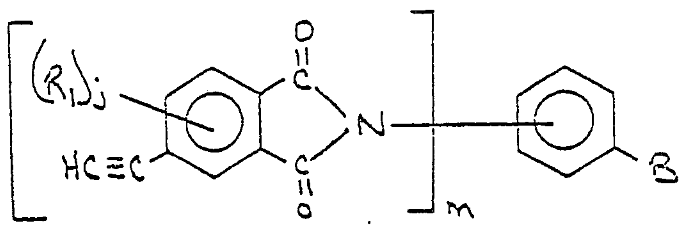

;

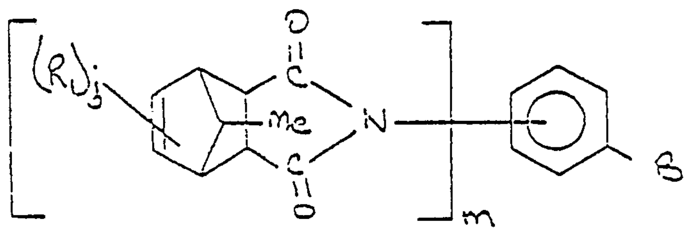

; and

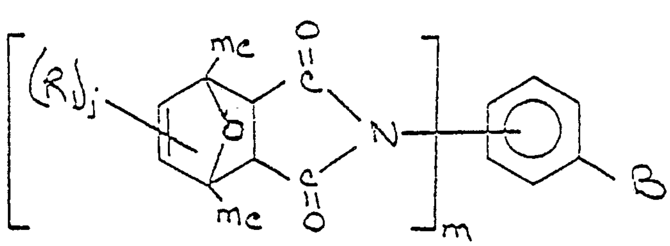

wherein B = X as defined for the hub,
provided that if X = CHO,

$B = -\overset{O}{\overset{\|}{C}}-Cl$;

m = 1 or 2;

$R_1$ = lower alkyl, lower alkoxy, aryl, and substituted aryl;

A = $-CH_2-$, -S-, -O-, or $-SO_2-$;

Me = $-CH_3$; and

j = 0, 1, or 2,
and with at least 3 moles of a solution containing an appropriate diamine, aldehyde, phenol, or mixture thereof under an inert atmosphere to form a compound having at least three arms, each arm including at least one Schiff base (-CH=N-) linkage to impart conductive or semiconductive properties to the doped oligomer.

13. The method of claim 12 wherein the end cap is selected from the group consisting of:

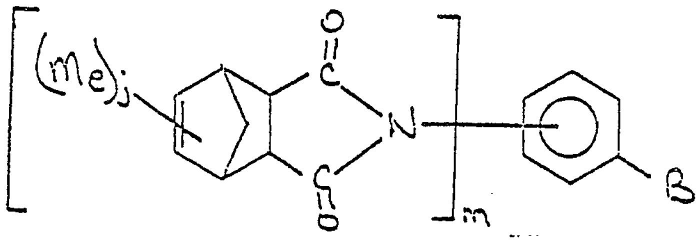

or

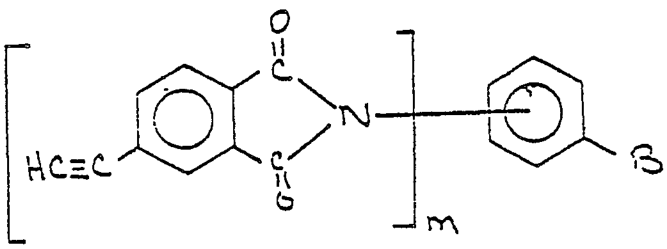

14. The method of claim 13 wherein the hub is

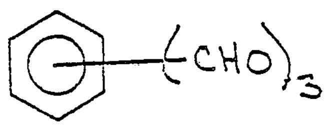

wherein

$B = Cl-\overset{O}{\overset{\|}{C}}-$, and

wherein the solution is a substantially equimolar mixture of:

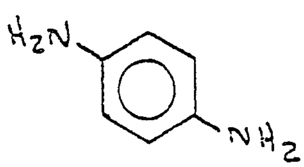

and

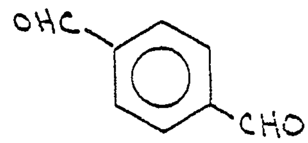

15. The method of claim 13 wherein the hub is

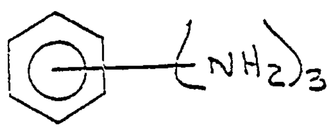

wherein B = $-NH_2$, and wherein the solution is

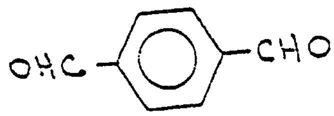

16. The method of claim 13 wherein the hub is

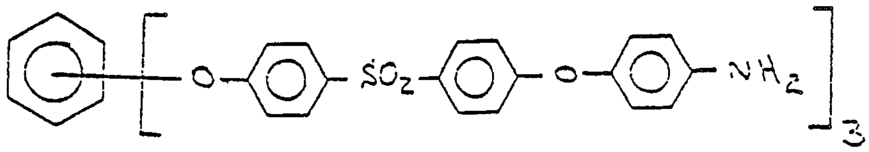

wherein B = $-NH_2$, and wherein the solution is

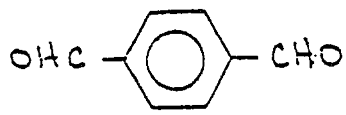

17. The method of claim 12 wherein Ar is selected from the group consisting of phenyl, biphenyl, naphthyl, and azalinyl.

18. A composite comprising the oligomer of claim 1 and suitable fiber reinforcement.

19. The composite of claim 18 further comprising an effective amount of a dopant in the oligomer, the dopant being selected from the group consisting of alkali metals, alkali perchlorates, arsenic compounds, elemental halogens, and mixtures thereof.

20. The composite of claim 19 wherein the fiber reinforcement is a fiber cloth.

21. A compound of the formula:

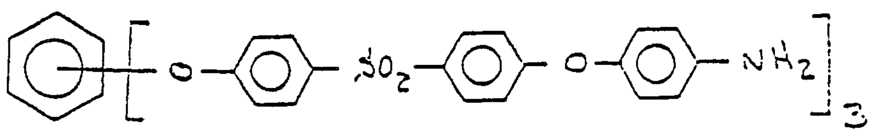

prepared by reacting p-aminophenol with 4,4′-dibromodiphenylsulfone and tribromobenzene under an inert atmosphere at an elevated temperature.

22. A prepreg comprising an oligomer selected from the group consisting of:

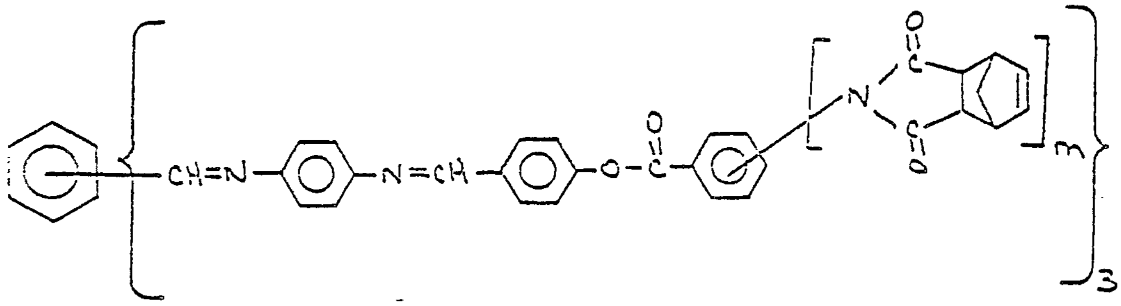

or

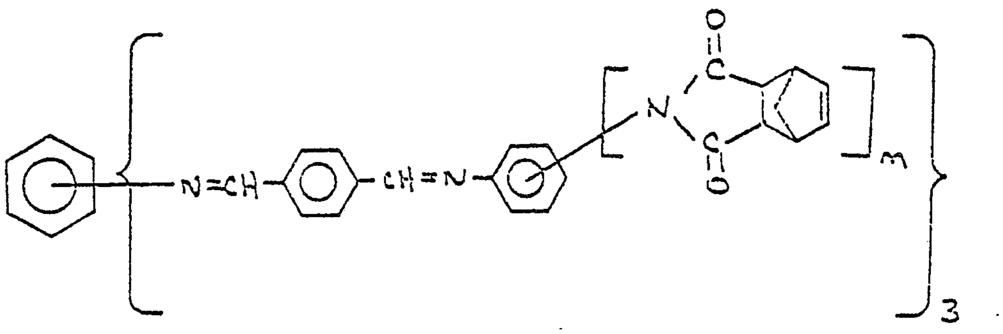

and a suitable fiber reinforcement.

23. The prepreg of claim 22 further comprising an effective amount of a dopant in the oligomer to make the oligomer conductive of semiconductive.

24. A composite formed by curing the prepreg of claim 23 at an elevated temperature in a conventional vacuum bag curing process.

25. A method for making a multidimensional oligomer, comprising the steps of:

(a) mixing one mole of

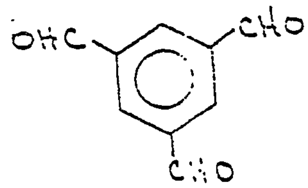

with about three moles of

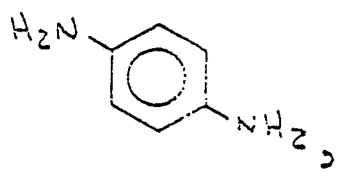

about three moles of

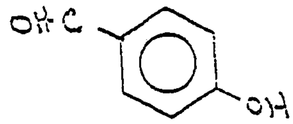

, and

about three moles of

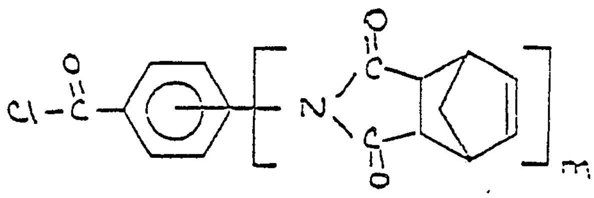

; and

(b) reacting the mixture under an inert atmosphere.

26. The product of the process of claim 25.

27. A method for making a multidimensional oligomer, comprising the steps of:

(a) mixing together one mole of

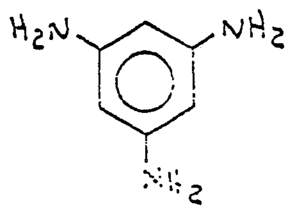

with about 3 moles of

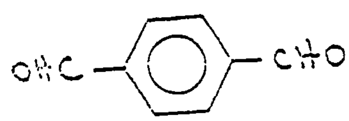

,and

about 3 moles of

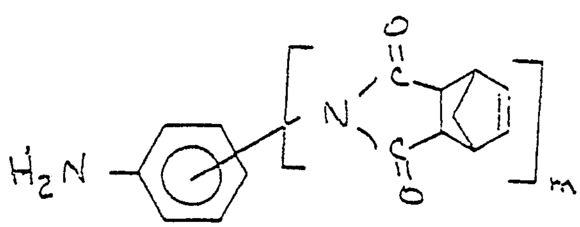

; and

(b) reacting the mixture under an inert atmosphere.

28. A conductive or semiconductive composite comprising an oligomer selected from the group consisting of the product of claim 25 or claim 27 and an effective amount of a dopant in the oligomer.

29. The composition of claim 28 wherein the dopant is selected from the group consisting of alkali metals, elemental halogen, alkali perchlorates, or mixtures thereof.

30. The composition of claim 29 further comprising fiber reinforcement in the oligomer.

31. An oligomer that is conductive or semiconductive when suitably doped with a conventional dopant, comprising the product of the process of reacting an aryl compound having three aldehyde functionalities with an aryl or polyaryl diamine having a formula weight of less than about 1000 and two terminal amine functionalities, with an aryl or polyaryl moiety having a formula weight of less than about 1000 and having two terminal aldehyde functionalities, and with an acid halide, crosslinking end cap phenylimide.

32. An oligomer that is conductive or semiconductive when suitably doped with a conventional dopant, comprising the product of the process of reacting an aryl compound having three amine functionalities with an aryl or polyaryl moiety having a formula weight less than about 1000 having two aldehyde functionalities and with an amine, crosslinking end cap phenylimide.

European Patent Office

# EUROPEAN SEARCH REPORT

Application Number

EP 88 10 0199

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 966 678 (M. GRUFFAZ et al.)<br>* whole document, in particular column 3, lines 31-58 * | 1,12 | C 07 D 209/48<br>C 07 D 209/76<br>C 07 D 207/44<br>C 07 D 519/00<br>C 08 J 5/24<br>H 01 B 1/12 |
| A,D | US-A-3 839 287 (G.T. KWIATKOWSKI et al.)<br>* claims * | 1 | |
| A | EP-A-0 148 534 (UNION CARBIDE CORP.)<br>* claims * | 1,22 | |
| A,D | US-A-4 476 184 (H.R. LUBOWITZ et al.) | | |
| A | EP-A-0 067 976 (THE BOEING CO.) | | |
| A | CHEMICAL ABSTRACTS; vol. 105, no. 8, 25th August 1986, page 27, abstract no. 61322q, Columbus, Ohio, US; C.H. SHEPPARD et al.: "Novel high-temperature matrix materials", & INT. SAMPE SYMP. EXHIB. 1986, 31, 1426-1433 | | |

TECHNICAL FIELDS SEARCHED (Int. Cl.4)

C 07 D 209/00
C 07 D 207/00
C 07 D 519/00
C 08 J 5/00
H 01 B 1/00

The present search report has been drawn up for all claims

| Place of search | Date of completion of the search | Examiner |
|---|---|---|
| BERLIN | 31-08-1988 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document